# EUROPÄISCHE PATENTSCHRIFT

(11) **EP 1 151 342 B1**
(45) Veröffentlichungstag und Bekanntmachung des Hinweises auf die Patenterteilung: **15.10.2003**
(21) Anmeldenummer: 00907485.7
(22) Anmeldetag: 28.01.2000
(51) Int. Cl.: G02B 23/24

(54) **VORRICHTUNG ZUM POSITIONIEREN ZUMINDEST EINES OPTISCHEN BAUELEMENTS INNERHALB EINES ENDOSKOPISCHEN SYSTEMS**
DEVICE FOR POSITIONING AT LEAST ONE OPTICAL STRUCTURAL PART WITHIN AN ENDOSCOPIC SYSTEM
DISPOSITIF POUR LE POSITIONNEMENT D'AU MOINS UN COMPOSANT OPTIQUE A L'INTERIEUR D'UN SYSTEME ENDOSCOPIQUE

(30) Priorität: 29.01.1999 DE 19903437
(43) Veröffentlichungstag der Anmeldung: 07.11.2001
(73) Patentinhaber: Karl Storz GmbH & Co. KG, 78532 Tuttlingen (DE)
(72) Erfinder: KEHR, Ulrich, D-70771 Leinfelden-Echterdingen (DE)
(74) Vertreter: Heuckeroth, Volker, Dr.
(86) Internationale Anmeldenummer: EP0000659
(87) Internationale Veröffentlichungsnummer: WO00045210

(56) Entgegenhaltungen:
- DE-A- 19 713 276
- US-A- 4 565 423
- US-A- 4 820 043

## Beschreibung

Die Erfindung betrifft eine Vorrichtung zum Positionieren zumindest eines optischen Bauelements innerhalb eines endoskopischen Systems, mit einem Gehäuse, durch das eine optische Achse des endoskopischen Systems verläuft und in dem das zumindest eine Bauelement angeordnet ist, wobei das Bauelement in den Strahlengang des endoskopischen Systems um eine Schwenkachse einschwenkbar und aus dem Strahlengang wieder ausschwenkbar ist, wobei die Schwenkachse schräg oder senkrecht zur optischen Achse verlaufend angeordnet ist, und wobei das zumindest eine Bauelement an einem Träger befestigt ist, der um die Schwenkachse verschwenkbar am Gehäuse befestigt ist.

Eine derartige Vorrichtung ist aus dem Dokument US 4,565,423 bekannt.

In dem zuvor genannten Dokument ist ein Endoskop beschrieben, das einen vom Endoskopgehäuse senkrecht wegführenden Lichtleiter aufweist, der zu einem Okular des Endoskops führt. Am Ende des Lichtleiters, das mit dem Endoskopgehäuse verbunden ist, sind mehrere optische Bauelemente an einem Träger angeordnet, der um eine senkrecht zur optischen Achse des Lichtleiters verlaufende Schwenkachse verschwenkbar ist, um jeweils eines der Bauelemente in den Strahlengang des endoskopischen Systems einzuschwenken oder aus diesem wieder auszuschwenken.

Unter optischen Bauelementen werden im Sinne der vorliegenden Erfindung beispielsweise Linsen, Filter, Blenden und dergleichen verstanden, die in einer Endoskopoptik verwendet werden.

Ein spezieller Anwendungsfall der vorliegenden Erfindung ist die Verwendung einer derartigen Vorrichtung in einem endoskopischen System für die photodynamische Diagnose, die photodynamische Therapie oder für die Fluoreszenzdiagnose.

Bei der photodynamischen Diagnose wird in einer Lichtquelle erzeugtes Licht einer bestimmten Spektralzusammensetzung endoskopisch in den Körper eingekoppelt und auf zu untersuchendes Gewebe gerichtet. Zuvor wird in das zu untersuchende Gewebe ein Photosensibilisator oder Markerstoff instilliert. Durch Bestrahlen des mit dem Photosensibilisator angereicherten Gewebes mit dem Anregungslicht wird eine lichtinduzierte Reaktion hervorgerufen, durch die von dem zu untersuchenden Gewebebereich Fluoreszenzstrahlung emittiert wird. Es gibt dazu Photosensibilisatoren, die sich bspw. in Tumorgewebe in stärkerem Maße anreichern als im gesunden Gewebe. Anhand der Intensitätsunterschiede der Fluoreszenzstrahlung wird es dadurch ermöglicht, Tumorgewebe von gesundem Gewebe kontrastreich zu differenzieren. Das Fluoreszenzlicht und das Anregungslicht liegen dabei in unterschiedlichen Spektralbereichen.

Um eine besonders kontrastreiche Beobachtung des zu untersuchenden Gewebes frei von einer die Beobachtung störenden Hintergrundstrahlung des Anregungslichtes, die das Beobachtungslicht überlagert, zu ermöglichen, wird in den Beobachtungsstrahlengang, d.h. in den Strahlengang des Fluoreszenzlichtes, ein Farbfilter gebracht, der eine hohe Transmission im Spektralbereich des Fluoreszenzlichtes aufweist, während seine Transmission im Spektralbereich des Beleuchtungs- oder. Anregungslichtes gering ist. Es werden häufig als Lichtquellen auch Weißlichtquellen verwendet, wobei in den Beleuchtungsstrahlengang ebenfalls ein Filter gebracht wird, der eine ausgeprägte Transmission im Spektralbereich des für die Anregung des Photosensibilisators geeigneten Spektralbereichs aufweist.

Unter Strahlengang im Sinne der vorliegenden Erfindung wird demnach der Strahlengang von Beleuchtungslicht, das sich von proximal nach distal ausbreitet, und/oder von Beobachtungslicht, das sich von distal nach proximal ausbreitet, verstanden.

Da mit demselben endoskopischen System nicht nur eine Beobachtung des Fluoreszenzlichtes möglich sein soll, sondern auch eine herkömmliche Beobachtung des Gewebebereiches mit Weißlicht, müssen solche Farbfilter nicht nur möglichst einfach in den Strahlengang einbringbar, sondern auch wieder herausnehmbar sein.

Es sind dazu Filter bekannt, die auf das Endoskop, bspw. auf dessen distales Ende, aufsteckbar sind. Solche Filter sind jedoch in der Handhabung umständlich. Zum Aufstecken bzw. Abnehmen eines solchen Filters ist es außerdem erforderlich, das Endoskop während der Diagnose oder Therapie aus dem Körper zu entnehmen, was die Untersuchung bzw. Behandlung des Patienten verlängert. Außerdem können solche aufsteckbaren Filter leicht verloren gehen.

Weiterhin ist aus dem Dokument DE-A-197 13 276 eine Vorrichtung zum Positionieren von Bauelementen innerhalb endoskopischer Systeme bekannt, die in einem Ausführungsbeispiel ein Revolverrad aufweist, das in dem Gehäuse um eine gehäusemittige Achse drehbar ist. Das Revolverrad trägt in einer Ebene verteilt mehrere optische Bauelemente, die um die mittige Achse als Schwenkachse in den Strahlengang des endoskopischen Systems geschwenkt werden können. Die Schwenkachse ist exzentrisch parallel zur optischen Achse verlaufend angeordnet. Diese Anordnung setzt jedoch voraus, daß die optische Achse des endoskopischen Systems exzentrisch zur Gehäusemittelachse angeordnet sein muß. Im Falle eines Endoskops bedeutet dies, daß das Gehäuse der Vorrichtung nicht konzentrisch zur Endoskopschaftachse angeordnet ist, was jedoch wünschenswert ist.

Würde man diese zuvor genannte Anordnung dahingehend abändern, daß das Gehäuse der Vorrichtung konzentrisch die optische Achse des endoskopischen Systems umgibt, müßte bei unverändertem Durchmesser des Revolverrades, der wegen der vorgegebenen Größe der optischen Bauelemente nicht verkleinert werden kann, das Gehäuse im Durchmesser fast auf das Doppelte vergrößert werden, wodurch der Nachteil einer radial sehr platzgreifenden Vorrichtung bestehen würde.

Der Erfindung liegt daher die Aufgabe zugrunde, eine Vorrichtung der eingangs genannten Art dahingehend weiterzubilden, daß ein Ein- und Ausschwenken des zumindest einen optischen Bauelements innerhalb des endoskopischen Systems ermöglicht wird, ohne daß die Vorrichtung radial raumgreifend baut.

Erfindungsgemäß wird diese Aufgabe hinsichtlich der eingangs genannten Vorrichtung dadurch gelöst, daß der Träger im Querschnitt senkrecht zur optischen Achse etwa L-förmig ausgebildet ist, wobei ein erster Schenkel des Trägers das zumindest eine Bauelement trägt, und ein zweiter Schenkel gelenkig am Gehäuse befestigt ist.

Während im Stand der Technik bei der aus der DE 197 13 276 A1 bekannten Vorrichtung an dem Konzept festgehalten wurde, die Schwenkachse, um die das zumindest eine optische Bauelement in den Strahlengang eingeschwenkt und aus diesem wieder ausgeschwenkt werden kann, parallel zur optischen Achse anzuordnen, ist demnach vorgesehen, die Schwenkachse schräg oder senkrecht zur optischen Achse verlaufend anzuordnen. Das Schwenken des zumindest einen optischen Bauelements um eine schräg oder senkrecht zur optischen Achse verlaufende Schwenkachse kommt einem Umklappen des Bauelements gleich. Das Ein- und Ausschwenken des Bauelements um eine parallel zur optischen Achse verlaufende Schwenkachse setzt nämlich stets voraus, daß die Schwenkachse exzentrisch zur optischen Achse verläuft, was stets eine radiale Mindestbaugröße der Vorrichtung erfordert. Bei einem Umklappen des einen oder der mehreren Bauelemente um eine schräg oder senkrecht zur optischen Achse verlaufende Schwenkachse dagegen kann eine radial schmalbauende Bauweise erreicht werden, weil das Bauelement keine Bewegung in Umfangsrichtung des Gehäuses ausführen muß. In der einfachsten Ausführungsform können ein oder mehrere Bauelemente einzeln in dem Gehäuse schwenkbar gelagert sein, bspw. drei Bauelemente in einer im Querschnitt gesehen dreieckigen Anordnung an axial gleicher Position, oder vier Bauelemente bspw. eine viereckige Anordnung an axial gleicher Position bilden, oder es können einzelne Bauelemente axial hintereinander um einzelne eigene Schwenkachsen verschwenkbar vorgesehen sein. Die erfindungsgemäße Ausgestaltung der Vorrichtung ermöglicht es besonders vorteilhaft, das Gehäuse, gleich ob in runder oder in eckiger Ausgestaltung, bezüglich der optischen Achse stets so anzuordnen, daß die optische Achse etwa mittig durch das Gehäuse hindurch geht, ohne daß es erforderlich ist, das Gehäuse dazu radial groß bauend ausgestalten zu müssen. Auf diese Weise können mehrere Bauelemente in dem Gehäuse angeordnet werden, die in platzsparender Weise jeweils einzeln oder zusammen in den Strahlengang des endoskopischen Systems eingeschwenkt und aus dem Strahlengang wieder ausgeschwenkt werden. Eine schräg oder senkrecht angeordnete Schwenkachse kann beispielsweise unter einem Winkel von 30°, 45°, 60° oder 90° oder Zwischenwinkeln davon zur optischen Achse angeordnet sein, wobei eine entsprechende Anordnung des optischen Bauelements zur Schwenkachse gewählt werden kann, wenn es aus Gründen der Minimierung der Reflexionsverluste und des Strahlenversatzes gewünscht ist, daß das optische Bauelement mit seinen lichtdurchtrittsseitigen Flächen senkrecht zur optischen Achse steht. Die erfindungsgemäße Vorrichtung kann vorteilhaft beispielsweise in ein Endoskop integriert sein, wobei dann das Gehäuse der Vorrichtung ein integraler Teil des Gehäuses des Endoskops ist, mit dem Vorteil, daß auch das Endoskop im Bereich der integrierten Vorrichtung radial schmalbauend ausgebildet werden kann.

Das zumindest eine Bauelement ist an einem Träger befestigt, der um die Schwenkachse verschwenkbar am Gehäuse befestigt ist, und der im Querschnitt etwa L-förmig ausgebildet ist, wobei ein erster Schenkel des Trägers das zumindest eine Bauelement trägt, und ein zweiter Schenkel gelenkig am Gehäuse befestigt ist.

Hierbei ist von Vorteil, daß mittels des Trägers eine stabile Halterung für das zumindest eine Bauelement bereitgestellt wird, die den Anforderungen an die Stabilität im Dauerbetrieb bei mehrmaligem Ein- und Ausschwenken genügt. Der Träger kann dabei das zumindest eine Bauelement in der Art einer Einfassung aufnehmen, wodurch das gegen mechanische Einflüsse empfindliche Bauelement randseitig geschützt ist. Die L-förmige Ausgestaltung des Trägers hat den Vorteil, daß sich dieser radial platzsparend um den Strahlengang herum in dem Gehäuse plazieren läßt, ohne den Lichtdurchgang im endoskopischen System störend zu beinflussen.

In einer bevorzugten Ausgestaltung ist die Schwenkachse senkrecht zur optischen Achse verlaufend angeordnet.

Diese Maßnahme hat den Vorteil, daß eine konstruktiv einfache Bauweise der Vorrichtung erreicht wird, weil bei einer etwa senkrecht zur optischen Achse verlaufenden Schwenkachse auch die relative Anordnung zwischen dem Bauelement und der Schwenkachse etwa rechtwinklig gewählt werden kann, insbesondere für den bereits zuvor erwähnten Fall, daß zur Minimierung von Reflexionsverlusten und des Strahlversatzes eine Positionierung des in den Strahlengang eingeschwenkten Bauelements senkrecht zur optischen Achse erreicht werden soll.

In einer weiteren bevorzugten Ausgestaltung ist die Schwenkachse so angeordnet, daß das Bauelement im aus dem Strahlengang ausgeschwenkten Zustand mit einer flächigen Seite einer Innenwand des Gehäuses benachbart zu liegen kommt.

Durch diese Maßnahme wird ein besonders platzsparender Ein- und Ausschwenkmechnismus für das zumindest eine Bauelement geschaffen. Das Gehäuse der Vorrichtung kann bei dieser Ausgestaltung radial so schmal bauend ausgebildet sein, daß zwischen dem für den Strahlengang innerhalb des endoskopischen Systems vorgesehenen lichten Querschnitt und der Innenwand des Gehäuses gerade ein Spalt verbleibt, in dem das zumindest eine Bauelement in seiner ausgeschwenkten Stellung, d.h. in seiner Ruheposition, noch Platz findet. In der Ruheposition liegt das Bauelement dann im wesentlichen parallel zur optischen Achse des endoskopischen Systems. Eine konstruktiv denkbar einfache Ausgestaltung besteht darin, die Schwenkachse an einem Rand des Bauelements anzuordnen und dieses in der Art einer Klappe in das Gehäuse einzubauen.

Weiterhin ist es bevorzugt, wenn in dem Gehäuse zumindest zwei schwenkbare Träger angeordnet sind, die jeweils zumindest ein Bauelement tragen und unabhängig voneinander verschwenkbar sind.

Hierbei ist von Vorteil, daß mit ein und derselben Vorrichtung wahlweise unterschiedliche Bauelemente, bspw. zwei oder mehrere optische Filter mit unterschiedlichen spektralen Transmissionseigenschaften, wahlweise oder gleichzeitig in den Strahlengang des endoskopischen Systems ein- und ausgeschwenkt werden können.

Dabei ist es bevorzugt, wenn die Träger miteinander derart gekoppelt sind, daß beim Einschwenken des zumindest einen Bauelements das zumindest eine andere Bauelement ausgeschwenkt wird und umgekehrt.

Hierbei ist von Vorteil, daß ein Schwenk- bzw. Klappmechanismus geschaffen wird, der eine vorteilhaft einfache Bedienung ermöglicht. Es kann bei dieser Ausgestaltung zwischen zwei oder mehreren Betriebszuständen durch einen einzigen Betätigungsmechanismus umgeschaltet werden, in denen jeweils bspw. ein Bauelement in den Strahlengang eingeschwenkt und die übrigen Bauelemente aus dem Strahlengang ausgeschwenkt sind.

In einer weiteren bevorzugten Ausgestaltung sind die Träger axial an einer etwa gleichen Position angeordnet.

Durch diese Maßnahme wird nicht nur eine radial, sondern auch axial schmal bauende Vorrichtung geschaffen. Insbesondere im Zusammenhang mit der zuvor erwähnten L-förmigen Ausgestaltung der Träger können zwei solcher Träger platzsparend symmetrisch zueinander in dem Gehäuse angeordnet und verschwenkbar gelagert werden.

Alternativ dazu ist es jedoch auch bevorzugt, wenn die Tragelemente an axial unterschiedlichen Positionen angeordnet sind.

Hierdurch wird der Vorteil erreicht, daß gleichzeitig zwei oder mehrere Bauelemente in den Strahlengang eingeschwenkt werden können. Z.B. kann der eine Träger eine Blende, der andere Träger einen Farbfilter und der dritte Träger einen Wärmeschutzfilter tragen, die axial hintereinander angeordnet gleichzeitig in den Strahlengang eingebracht werden können, was je nach Verwendung des endoskopischen Systems sinnvoll sein kann.

In einer weiteren bevorzugten Ausgestaltung trägt der Träger in Schwenkrichtung umfänglich verteilt mehrere Bauelemente.

Bei dieser Ausgestaltung ist nur ein verschwenkbarer Träger vorgesehen, mit dem wahlweise einzelne optische Bauelemente in den Strahlengang eingeschwenkt und aus dem Strahlengang wieder ausgeschwenkt werden können, wodurch vorteilhafterweise mit nur einem Träger mehrere Bauelemente verschwenkbar in dem Gehäuse angeordnet werden können, was vorteilhafterweise eine Reduzierung der Zahl der Teile der Vorrichtung bedeutet. Bei einer derartigen Ausgestaltung des Trägers wird ebenfalls die bereits zuvor erwähnte Schwenkkopplung erreicht, derart, daß beim Einschwenken des zumindest einen Bauelements das weitere oder die weiteren Bauelemente automatisch aus dem Strahlengang ausgeschwenkt werden.

In einer weiteren bevorzugten Ausgestaltung ist zum Betätigen des Ein- und Ausschwenkens des zumindest einen Bauelements eine Magnetkupplung vorgesehen, die zumindest ein außerhalb des Gehäuses angeordnetes äußeres bewegliches magnetisch wirksames Element oder einen Magneten und zumindest ein innerhalb des Gehäuses angeordnetes inneres magnetisch wirksames Element aufweist, wobei das äußere magnetisch wirksame Element oder der Magnet und das innere magnetisch wirksame Element durch das Gehäuse hindurch über einen magnetischen Kraftschluß zusammenwirken.

Diese bei derartigen Vorrichtungen an sich bekannte Magnetkupplung hat wiederum den Vorteil, daß das Gehäuse der Vorrichtung hermetisch dicht geschlossen ausgebildet werden kann, wodurch der Vorteil erreicht wird, daß die Vorrichtung und damit das endoskopische System, an dem die Vorrichtung vorgesehen ist, den Bedingungen in einem Autoklaven standhalten können, so daß die für medizinische Zwecke geforderte Reinigbarkeit der Vorrichtung gewährleistet wird. Sofern im folgenden von äußeren Magneten gesprochen wird, können diese auch durch magnetisch wirksame Elemente aus magnetischen Werkstoffen, wie Weicheisenkerne, ersetzt werden, zumindest dann, wenn als innere magnetisch wirksame Elemente Magnete vorgesehen sind.

In einer weiteren bevorzugten Ausgestaltung ist der zumindest eine Träger als bezüglich der Schwenkachse zweiarmiger Hebel ausgebildet, dessen einer Hebelarm das zumindest eine Bauelement trägt, und an dessen anderem Hebelarm ein im wesentlichen axial bewegliches Kraftübertragungselement angreift.

In Verbindung mit der schräg zur optischen Achse verlaufenden Schwenkachse ergibt sich durch diesen Hebelmechanismus mit einem axial beweglichen Kraftübertragungselement im Zusammenhang mit dem als zweiarmigen Hebel ausgebildeten Träger ein konstruktiv einfacher Betätigungsmechanismus für das Ein- und Ausschwenken des zumindest einen Bauelements.

Dabei ist es weiterhin bevorzugt, wenn der zumindest eine äußere Magnet und das zumindest eine innere magnetisch wirksame Element der zuvor erwähnten Magnetkupplung axial verschieblich sind, wobei das innere magnetisch wirksame Element mit dem Kraftübertragungselement verbunden ist.

Diese Ausgestaltung der Magnetkupplung weicht insofern von der Magnetkupplung der bekannten Vorrichtung ab, als bei letzterer sowohl der zumindest eine äußere Magnet als auch der zumindest eine innere Magnet drehbar, jedoch axial unverschieblich ausgebildet sind. Im Unterschied dazu wird bei der vorliegenden Ausgestaltung der Magnetkupplung der Vorteil erreicht, daß im Zusammenhang mit dem zuvor erwähnten Hebelmechanismus der Bewegungsübertragungsmechanismus von dem äußeren Magneten als Stellglied auf den Hebelmechanismus konstruktiv besonders einfach ausgestaltet ist, weil keine mechanischen Bauteile in der Vorrichtung vorgesehen sein müssen, um wie bei der bekannten Vorrichtung eine Drehbewegung der Magnete in eine axiale Bewegung des Hebelmechanismus umzusetzen. Dadurch wird der konstruktive Aufwand der erfindungsgemäßen Vorrichtung wesentlich verringert.

In einer weiteren bevorzugten Ausgestaltung weist die Magnetkupplung zumindest zwei äußere Magnete auf, die an axial unterschiedlichen Positionen an einem drehbaren Ring angeordnet sind, wobei das zumindest eine innere magnetisch wirksame Element axial verschieblich und mit dem Kraftübertragungselement verbunden ist, und wobei die zwei äußeren Magnete durch Drehen des Rings alternativ mit dem inneren Element magnetisch in Eingriff bringbar sind.

Hierbei ist von Vorteil, daß weiterhin eine konstruktiv einfache Magnetkupplung geschaffen wird, bei der weiterhin keine mechanischen Bauteile vorgesehen sein müssen, um eine Drehbewegung in eine axiale Bewegung umzusetzen. Durch das leicht bedienbare Drehen des äußeren drehbaren Ringes, der bevorzugt axial unverschieblich um das Gehäuse herum angeordnet ist, wird durch alternatives in Eingriff bringen einer der beiden äußeren Magneten mit dem einen inneren magnetisch wirksamen Element eine axiale Hin- und Herbewegung des inneren magnetisch wirksamen Elements bewirkt, weil die beiden äußeren Magnete an axial verschiedenen Positionen angeordnet sind.

In einer weiteren bevorzugten Ausgestaltung ist das zumindest eine innere magnetisch wirksame Element am Träger selbst angeordnet und wirkt mit dem zumindest einen äußeren Magneten zum Ein- und Ausschwenken des zumindest einen Bauelements unmittelbar zusammen.

Bei dieser Ausgestaltung der Magnetkupplung besteht der Vorteil, daß zum Umklappen des Trägers, der das zumindest eine Bauelement trägt, keine weiteren Kraftübertragungselemente erforderlich sind, wodurch der Aufbau der Magnetkupplung konstruktiv weiter vereinfacht und darüber hinaus die Gefahr von Funktionsstörungen aufgrund der geringeren Anzahl beweglicher Teile verringert wird.

Dabei ist es wiederum bevorzugt, wenn der zumindest eine äußere Magnet über einen das Gehäuse umgebenden drehbaren Ring beweglich ist.

Durch diese Maßnahme wird wiederum ein einfacher Betätigungsmechanismus für die Magnetkupplung geschaffen, mit dem das zumindest eine Bauelement auf einfach zu bedienende Weise in den Strahlengang ein- und aus dem Strahlengang ausgeschwenkt werden kann.

In einer weiteren bevorzugten Ausgestaltung sind zwei innere magnetisch wirksame Elemente in Form von zwei Magneten am Träger angeordnet, wobei die inneren Magnete bezüglich der Schwenkachse einander gegenüberliegend angeordnet und entgegengerichtet polarisiert sind, wobei zumindest zwei äußere Magnete außerhalb des Gehäuses angeordnet sind, die wechselweise in eine Stellung bewegbar sind, in der sie mit den inneren Magneten magnetisch zusammenwirken, um das zumindest eine Bauelement einund auszuschwenken.

Dies stellt eine bevorzugte und vorteilhafte Ausgestaltung einer Magnetkupplung mit unmittelbar am Träger angeordneten magnetischen Elementen dar, bei der das Umklappen des Trägers zum Ein- und Ausschwenken des zumindest einen Bauelements durch zumindest zwei entgegengesetzt gerichtete Magnetfelder bewerkstelligt wird. Aufgrund der bezüglich der Schwenkachse gegenüberliegenden Anordnung der beiden inneren Permanentmagnete und deren entgegengerichteter Polarisierung entsteht jedesmal, wenn der zumindest eine andere der äußeren Magnete mit den beiden inneren Permanentmagneten magnetisch in Eingriff gebracht wird, ein Drehmoment, das den Träger aus seiner vorherigen Position, bspw. der eingeschwenkten Position, in die andere Position, d.h. dann in die ausgeschwenkte Position, bewegt.

Bei den zuvor erwähnten Ausgestaltungen, bei denen der zumindest eine äußere Magnet an einem drehbaren Ring angeordnet ist, der als Betätigungs- bzw. Bedienelement zum Ein- und Ausschwenken des zumindest einen Bauelements dient, ist es weiterhin bevorzugt, wenn dieser Ring zumindest zwei Raststellungen aufweist, wobei zumindest eine Raststellung einer ausgeschwenkten Stellung des Bauelements und zumindest eine Raststellung einer eingeschwenkten Stellung des Bauelements zugeordnet ist.

Anhand solcher Raststellungen, in denen der Ring hörbar oder fühlbar einrastet, kann der Benutzer feststellen, wie weit er den Ring drehen muß, um von der eingeschwenkten Stellung des zumindest einen Bauelements in die ausgeschwenkte Stellung zu gelangen. Bei einer Anbringung einer entsprechenden Markierung am Ring kann er außerdem stets die Stellung des zumindest einen Bauelements feststellen.

In einer weiteren bevorzugten Ausgestaltung ist der zumindest eine äußere Magnet ein Permanentmagnet.

Die Verwendung eines Permanentmagneten hat den Vorteil, daß die Magnetkupplung besonders einfach aufgebaut werden kann, insbesondere werden keine Stromzuführungen wie für Elektromagneten benötigt.

In einer weiteren bevorzugten Ausgestaltung ist der zumindest eine äußere Magnet ein Elektromagnet, wobei das zumindest eine innere magnetisch wirksame Element gegebenenfalls mit einer Rückholfeder verbunden ist.

Auch die Verwendung eines Elektromagneten für die Magnetkupplung ist vorteilhaft, weil das Ein- und Ausschwenken des optischen Bauelements durch Umschalten der Stromrichtung in der Elektrospule betätigt werden kann, ohne daß dazu ein äußeres Stellglied am Gehäuse der Vorrichtung vorgesehen werden muß. Gegebenenfalls kann das innere magnetisch wirksame Element mit einer Rückholfeder verbunden und dadurch in eine Endstellung vorgespannt sein, so daß das Ein- und Ausschwenken des Bauelements nicht durch Umschalten der Stromrichtung, sondern allein durch Ein- und Ausschalten der Stromzuführung in die Elektrospule betätigt werden kann.

In einer weiteren bevorzugten Ausgestaltung ist das zumindest eine innere magnetisch wirksame Element ein Magnet oder ein Weicheisenkern.

In einer weiteren bevorzugten Ausgestaltung ist im Innern des Gehäuses ein Tauchspulantrieb mit einer Elektrospule und einem darin axial beweglichen Anker angeordnet, wobei der Anker mit dem Kraftübertragungselement verbunden ist.

Bei dieser Ausgestaltung ist demnach keine von außen durch das Gehäuse nach innen wirkende Magnetkupplung vorgesehen, sondern als Betätigungseinrichtung zum Ein- und Ausschwenken des optischen Bauelements ist im Inneren eine Tauchspule, d.h. eine Elektrospule angeordnet, in der ein Anker, beispielsweise ein Magnet oder Weicheisenkern, axial beweglich angeordnet ist. Die Elektrospule muß dann allerdings von außen durch das Gehäuse hindurch mit Strom beaufschlagt werden. Durch Umschalten der Stromrichtung wird dann der mit dem Kraftübertragungselement verbundene Anker axial hin- und herbewegt, wodurch dann das optische Bauelement ein- und ausgeschwenkt wird. Auch hier kann wiederum der Anker mit einer Rückholfeder verbunden sein, so daß die Betätigung des optischen Bauelements durch Ein- und Ausschalten des Stroms bewirkt wird. Der Vorteil dieser Ausgestaltung besteht in einer weiteren Reduzierung der radialen Abmessung der Vorrichtung, da ein äußeres Betätigungselement am Gehäuse der Vorrichtung nicht notwendig ist. Im Innern kann auch eine Doppelspule mit je einer Wicklung für jede Stellung des optischen Bauelements vorgesehen sein, wobei dann durch entsprechende Ansteuerung der entsprechenden Wicklung das Einund Ausschwenken des optischen Bauelements betätigt wird.

Ein erfindungsgemäßes Endoskop, das insbesondere für die photodynamische Diagnose, Therapie oder die Fluoreszenzdiagnose verwendet wird, weist eine erfindungsgemäße Vorrichtung der zuvor beschriebenen Art bzw. vorbeschriebenen Arten auf.

Dabei ist es bevorzugt, wenn die Vorrichtung am proximalen Ende des Endoskops in einem Optikkopf zwischen der Okularlinse und dem Deckglas des Okulars angeordnet ist.

Hierbei ist von Vorteil, daß an dieser Stelle des Endoskops genug Platz für die Vorrichtung vorhanden ist, und daß das optische Bauelement, beispielsweise ein Filter, weit genug von Zwischenbildebenen entfernt liegt, so daß etwaige Unsauberkeiten des Bauelements, beispielsweise Staubpartikel, nicht abgebildet werden.

In einer weiteren bevorzugten Ausgestaltung bildet das Gehäuse der Vorrichtung das Gehäuse des Endoskops.

Diese Maßnahme hat den Vorteil, daß das Gehäuse der Vorrichtung integraler Bestandteil des Endoskopgehäuses ist, wodurch es ermöglicht wird, das Endoskopgehäuse insgesamt hermetisch dicht und außerdem das Endoskopgehäuse selbst radial schmalbauend auszubilden.

In einer weiteren bevorzugten Ausgestaltung ist das Gehäuse hermetisch dicht.

Hierbei ist von Vorteil, daß sich das Endoskop in einem Autoklaven sterilisieren läßt, ohne daß in das Innere des Gehäuses Feuchtigkeit oder Verunreinigungen eindringen können. Die hermetisch dichte Ausgestaltung des Gehäuses wird einerseits durch die zuvor erwähnte Magnetkupplung, andererseits durch die mit dem Gehäuse des Endoskops integrale Bauweise der Vorrichtung erzielt.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf diese näher beschrieben. Es zeigen:
- Fig. 1: eine schematische Gesamtdarstellung eines Endoskops;
- Fig. 2: einen Querschnitt durch eine Vorrichtung zum Positionieren zumindest eines optischen Bauelements innerhalb des Endoskops;
- Fig. 3a) und 3b): einen Schnitt durch die Vorrichtung in Fig. 2 entlang der Linie III-A-B-C-D-III in Fig. 2, wobei Fig. 3a) die Vorrichtung in einer ersten Betriebsstellung und die Fig. 3b) die Vorrichtung in einer zweiten Betriebsstellung zeigt;
- Fig. 4: einen Fig. 3a) bzw. 3b) entsprechenden Schnitt durch eine weitere Vorrichtung gemäß einem weiteren Ausführungsbeispiel;
- Fig. 5: einen Fig. 2 entsprechenden Querschnitt durch eine weitere Vorrichtung gemäß einem noch weiteren Ausführungsbeispiel;
- Fig. 6a) bis 6d): eine Vorrichtung zum Positionieren zumindest eines Bauelements gemäß einem weiteren Ausführungsbeispiel in schematisch vereinfachten perspektivischen Darstellungen, die die Funktionsweise der Vorrichtung veranschaulichen; und
- Fig. 7a) und 7b): Fig. 6c) und 6d) entsprechende Darstellungen eines gegenüber Fig. 6 geringfügig abgewandelten Ausführungsbeispiels einer Vorrichtung zum Positionieren zumindest eines optischen Bauelements.

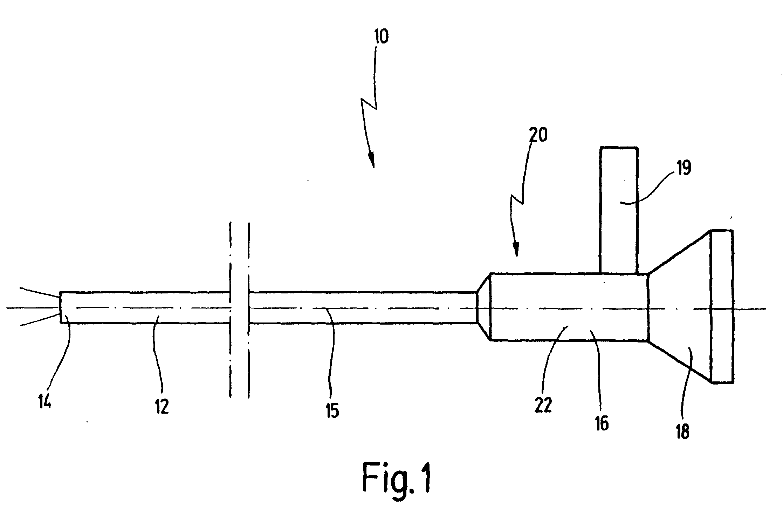

In Fig. 1 ist ein mit dem allgemeinen Bezugszeichen 10 versehenes Endoskop als endoskopisches System für die photodynamische Diagnose bzw. für die Fluoreszenzdiagnose dargestellt.

Das Endoskop 10 weist einen lang erstreckten Schaft 12 auf, in dem ein nicht näher dargestelltes optisches Abbildungssystem aus mehreren hintereinander angeordneten Linsen enthalten ist. Ein distales Ende 14 des Schafts 12 bildet das lichtaustrittsseitige Ende für Beleuchtungslicht sowie das lichteintrittsseitige Ende für Beobachtungslicht des endoskopischen Systems.

Eine optische Achse 15 des optischen Systems fällt etwa mit der Längsmittelachse des Schafts 12 zusammen. An ein proximales Ende des Schafts 12 schließt sich ein Optikkopf 16 an, der an seinem proximalen Ende eine Okularmuschel 18 eines Okulars trägt.

Ein Lichtleiteranschluß 19 dient zum Anschließen eines nicht dargestellten Lichtleitkabels, um in einer externen nicht dargestellten Lichtquelle erzeugtes Licht in das Endoskop 10 einzukoppeln, das dann am distalen Ende 14 zur Beleuchtung eines Untersuchungsareals im menschlichen oder tierischen Körper austritt.

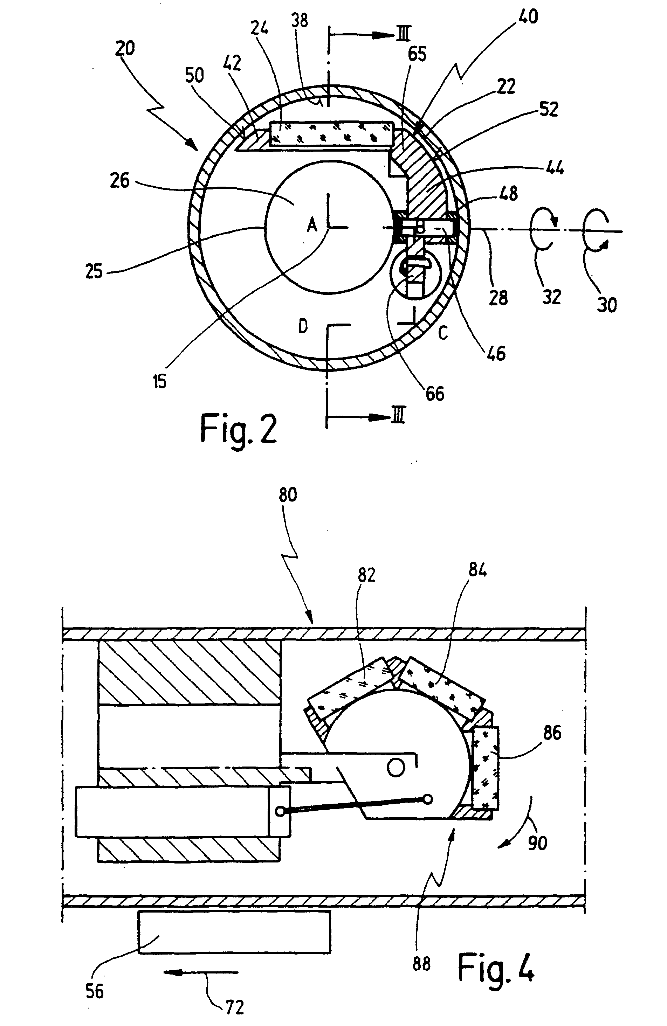

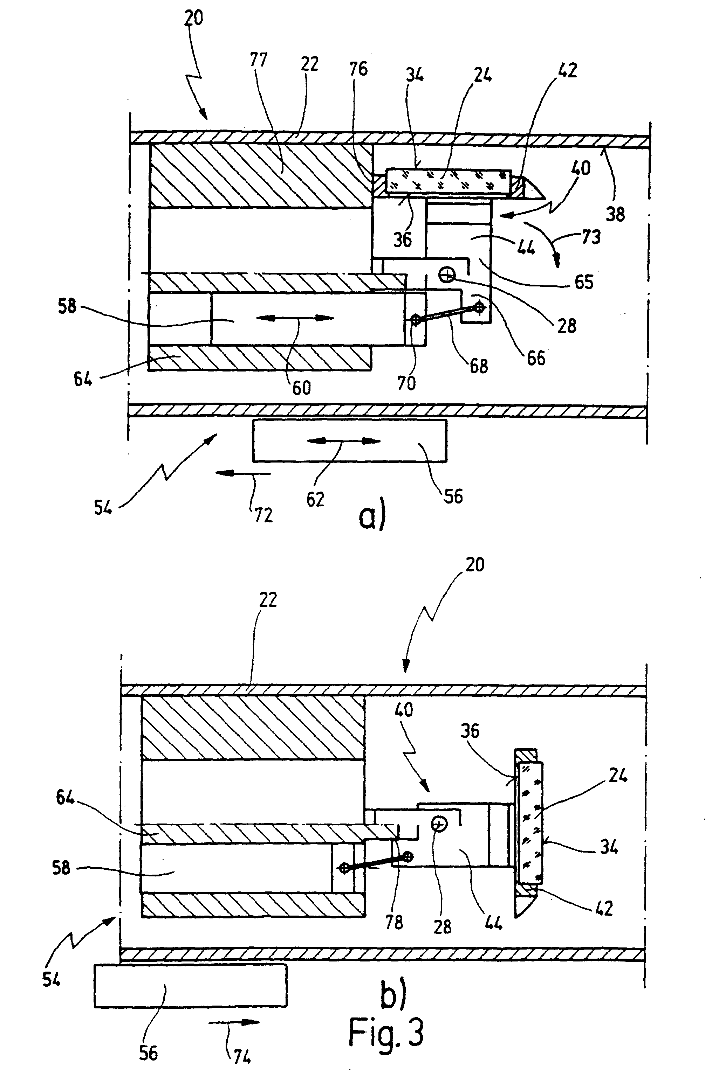

Das Endoskop 10 weist eine erfindungsgemäße Vorrichtung 20 zum Positionieren zumindest eines optischen Bauelements innerhalb des Endoskops 10 auf, die hiernach mit Bezug auf Figuren 2 und 3 näher beschrieben wird.

Die Vorrichtung 20 weist ein Gehäuse 22 auf, das im gezeigten Ausführungsbeispiel zylindrisch ausgebildet ist. Das Gehäuse 22 bildet gleichzeitig das Gehäuse des Optikkopfes 16 des Endoskops 10 in Fig. 1 und ist somit integraler Bestandteil des Gehäuses des Endoskops 10.

In dem Gehäuse 22 ist ein optisches Bauelement 24, bspw. ein optisches Filter, angeordnet. Die optische Achse 15 in Fig. 1 verläuft etwa mittig durch das Gehäuse 22.

Ein in Fig. 2 eingezeichneter Kreis 25, der konzentrisch zu dem Gehäuse 22 ist, grenzt eine Querschnittsfläche 26 ein, die die Querschnittsfläche des Strahlengangs des Endoskops 10 darstellt.

Das optische Bauelement 24 ist um eine Schwenkachse 28 in den Strahlengang des Endoskops 10 gemäß einem Pfeil 30 einschwenkbar und gemäß einem Pfeil 32 aus dem Strahlengang ausschwenkbar. Fig. 2 bzw. Fig. 3a) zeigen das optische Bauelement 24 im aus dem Strahlengang ausgeschwenkten Zustand, während Fig. 3b) das optische Bauelement 24 im in den Strahlengang eingeschwenkten Zustand zeigt.

Der hier betrachtete Strahlengang kann sowohl der Strahlengang des Beleuchtungslichtes, dessen Lichtweg von proximal nach distal führt, als auch der Strahlengang des Beobachtungslichtes sein, dessen Lichtweg von distal nach proximal führt.

Im in den Strahlengang eingeschwenkten Zustand verlaufen lichtdurchtrittsseitige Endflächen 34 bzw. 36 des optischen Bauelements 24 schräg zum Strahlengang, d.h. schräg zur optischen Achse 15, und überdecken dabei im wesentlichen die Querschnittsfläche 26 des Strahlengangs.

Im aus dem Strahlengang ausgeschwenkten Zustand gemäß Fig. 3a) liegt die lichtdurchtrittsseitige Endfläche 34 einer Innenwand 38 des Gehäuses 22 benachbart. Im ausgeschwenkten Zustand ist das optische Bauelement 24 vollständig aus dem Strahlengang des Endoskops 10 entfernt.

Wie aus Fig. 2 und Figuren 3a) und 3b) hervorgeht, verläuft die Schwenkachse 28 schräg zur optischen Achse 15. Im gezeigten Ausführungsbeispiel verläuft die Schwenkachse 28 etwa senkrecht zur optischen Achse 15. Ferner liegt die Schwenkachse 28 auf einer gedachten Geraden, die die optische Achse 15 schneidet. Ferner liegt die Schwenkachse 28 auf einem Durchmesser des Gehäuses 22.

Im aus dem Strahlengang ausgeschwenkten Zustand befindet sich das Bauelement 24 im Querschnitt gesehen zwischen der Querschnittsfläche 26 des Strahlengangs und der Innenwand 38 des Gehäuses 22.

Das Bauelement 24 ist an einem Träger 40 befestigt. Der Träger 40 ist im Querschnitt gemäß Fig. 2 etwa L-förmig ausgebildet. Der Träger 40 weist dazu einen ersten Schenkel 42 auf, der das Bauelement 24 in der Art einer Einfassung trägt. Ein zweiter Schenkel 44, der mit dem ersten Schenkel 42 einstückig verbunden ist, verläuft im wesentlichen rechtwinklig zu dem ersten Schenkel 42.

Der zweite Schenkel 44 ist mittels eines Gelenkzapfens 46, der in einer Lagergabel 48 drehbar aufgenommen ist, gelenkig mit dem Gehäuse 22 verbunden.

Aufgrund der L-förmigen Ausgestaltung des Trägers 40 wird gewährleistet, daß die Schenkel 42 bzw. 44 sowohl im eingeschwenkten Zustand des Bauelements 24 gemäß Fig. 3b) als auch im ausgeschwenkten Zustand gemäß Fig. 2 bzw. Fig. 3a) kein Lichthindernis darstellen. Insbesondere der zweite Schenkel 44 bewegt sich beim Ein- und Ausschwenken des Bauelements 24 stets entlang der Innenwand 38 des Gehäuses 22, ohne durch den Strahlengang hindurchzutreten. Außenseiten 50 des ersten Schenkels 42 bzw. 52 des zweiten Schenkels 44 sind zur weiteren Platzersparnis an die Kontur der Innenwand 38 des Gehäuses 22 angepaßt.

Zum Betätigen des Ein- und Ausschwenkens des Bauelements 24 in den Strahlengang und aus dem Strahlengang weist die Vorrichtung 20 ferner eine Magnetkupplung 54 auf.

Die Magnetkupplung 54 weist zumindest einen außerhalb des Gehäuses 22 angeordneten äußeren beweglichen Magneten 56 auf, der in Figuren 3a) und 3b) nur äußerst schematisch dargestellt ist.

Die Magnetkupplung 54 weist weiterhin zumindest einen innerhalb des Gehäuses 22 angeordnetes inneres bewegliches magnetisch wirksames Element 58 auf, das ebenfalls nur schematisch dargestellt ist. Der zumindest eine äußere Magnet 56 und das zumindest eine innere magnetisch wirksame Element 58 wirken durch das Gehäuse 22 hindurch über einen magnetischen Kraftschluß zusammen, d.h. eine Bewegung des äußeren Magneten 56 bewirkt eine gleichgerichtete Bewegung des inneren Elements 58. Dadurch, daß zum Betätigen des Ein- und Ausschwenkens des Bauelements 24 eine Magnetkupplung 54 vorgesehen ist, ist das Gehäuse 22 bevorzugt hermetisch dicht ausgebildet, d.h. es weist keine Öffnungen auf, die im Fall eines rein mechanisch wirkenden Betätigungsmechanismus vorgesehen sein müßten, um eine Bewegung eines außerhalb des Gehäuses 22 angeordneten Stellgliedes durch das Gehäuse 22 hindurch auf ein in dem Gehäuse 22 angeordnetes mechanisches Stellglied oder Kraftübertragungselement zu übertragen.

Der zumindest eine äußere Magnet 56 und das zumindest eine innere magnetisch wirksame Element 58 sind gemäß Doppelpfeilen 60 und 62 magnetisch miteinander gekoppelt axial beweglich.

Das innere Element 58 ist dazu in einer Führungshülse 64 aufgenommen, die außerhalb der Querschnittsfläche 26 des Strahlengangs angeordnet ist, so daß der Strahlengang durch die Führungshülse 64 nicht beeinträchtigt wird. Der dem inneren Element 58 gegenüberliegende äußere Magnet 56 kann auf der Innenseite einer verschiebbar um das Gehäuse 22 herum angeordneten Hülse befestigt sein, wobei dann diese Hülse, die im einzelnen nicht dargestellt ist, als Bedienungselement für den Benutzer in Form eines Schiebers dient.

Das zumindest eine innere magnetisch wirksame Element 58 ist beispielsweise ein Magnet in Form eines Permanentmagneten oder ein Weicheisenkern.

Der zumindest eine äußere Magnet 56 ist ein Permanentmagnet, kann jedoch auch ein Elektromagnet sein.

Anstelle der zuvor genannten Anordnung, bei der der zumindest eine äußere Magnet 56 und das zumindest eine innere magnetisch wirksame Element 58 axial verschieblich sind, kann die Magnetkupplung jedoch auch so ausgebildet sein, daß sie zumindest zwei äußere Magneten aufweist, die an axial unterschiedlichen Positionen etwa diametral gegenüberliegend an einem drehbaren Ring angeordnet sind, wobei das zumindest eine innere magnetisch wirksame Element 58 weiterhin axial verschieblich ist, wobei dann die zwei äußeren Magnete durch Drehen des Rings wahlweise mit dem inneren Element 58 in Eingriff bringbar sind, und zwar abwechselnd.

Der Träger 40 ist weiterhin als zweiarmiger Hebel ausgebildet, wobei ein erster Hebelarm 65, der durch die Schenkel 44 und 42 gebildet wird, das Bauelement 24 trägt, und an dessen anderem Hebelarm 66 ein im wesentlichen axial bewegliches Kraftübertragungselement 68 in Form einer Zug- und Druckstange angelenkt ist. Das Kraftübertragungselement 68 ist dazu mit dem dem zweiten Hebelarm 66 gegenüberliegenden Ende 70 an dem inneren magnetisch wirksamen Element 58 angelenkt. Auf diese Weise wird ein Hebelmechanismus zum Verschwenken des Trägers 40 und damit des Bauelements 24 um die Schwenkachse 28, die durch den Gelenkzapfen 46 gebildet wird, geschaffen.

Ausgehend von Fig. 3a), die das Bauelement 24 im aus dem Strahlengang ausgeschwenkten Zustand zeigt, wird durch Bewegen des äußeren Magneten 56 in Richtung eines Pfeiles 72 auch das innere Element 58 in Richtung des Pfeiles 72 bewegt, wobei die Bewegung des inneren Element 58 über das Kraftübertragungselement 68 auf den zweiten Hebelarm 66 des Trägers 40 übertragen wird, wodurch der Träger 40 in Richtung eines Pfeiles 73 in die in Fig. 3b) dargestellte Lage verschwenkt wird, in der das Bauelement 24 in den Strahlengang des Endoskops 10 eingeschwenkt ist. Ausgehend von der Stellung des Bauelements 24 in Fig. 3b) wird dieses durch Bewegen des äußeren Magneten 56 in Richtung des Pfeiles 74 wieder aus dem Strahlengang ausgeschwenkt.

Im Falle der zuvor erwähnten Ausgestaltung der Magnetkupplung, bei der zumindest zwei äußere Magneten vorgesehen sind, die mit dem zumindest einen inneren axial beweglichen magnetisch wirksamen Element 58 zusammenwirken, wobei die beiden äußeren Magnete an axial verschiedenen Positionen und etwa diametral gegenüberliegend angeordnet sind, ergibt sich folgende Funktionsweise. Wird durch Drehen des Rings der axial rechte Magnet mit dem inneren Element 58 magnetisch in Eingriff gebracht, wird das innere Element 58 gemäß dem Pfeil 60 nach rechts bewegt, wodurch das Bauelement 24 aus dem Strahlengang geklappt wird. Wird durch weiteres Drehen des Rings um etwa 180° der linke äußere Magnet mit dem inneren Element 58 magnetisch in Eingriff gebracht, wird das innere Element 58 gemäß dem Pfeil 60 in Fig. 3a) nach links verschoben, wodurch das Bauelement 24 in den Strahlengang klappt. Durch Drehen des Rings kann somit zwischen den zwei Schwenkendstellungen gemäß Fig. 3a) und 3b) umgeschaltet werden.

Eine andere Ausgestaltung der zuvor beschriebenen Magnetkupplung kann darin bestehen, daß der äußere Magnet anstelle in Form eines Permanentmagneten in Form eines Elektromagneten ausgebildet ist, d.h. eine Elektrospule aufweist, die entsprechend mit Strom beaufschlagt wird. Durch Umschalten der Stromrichtung kann dann das innere magnetisch wirksame Element 58 zum Einund Ausschwenken des optischen Bauelements 24 hin- und herbewegt werden. Das innere magnetisch wirksame Element 58 kann auch in eine seiner Endstellungen durch eine Rückholfeder vorgespannt sein, so daß ein Hin- und Herbewegen des Elements 58 durch Ein- und Ausschalten des Stroms in der Elektrospule bewirkt wird.

Anstelle der zuvor beschriebenen Magnetkupplungen kann alternativ auch ein in dem Gehäuse 22 angeordneter Tauchspulantrieb vorgesehen sein, der eine Tauchspule, d.h. eine Elektrospule aufweist, in der ein Anker, beispielsweise ein Magnet oder ein Weicheisenkern axial beweglich angeordnet ist. Der Anker ist dann mit dem Kraftübertragungselement 68 kraftschlüssig verbunden. Durch eine entsprechende Strombeaufschlagung kann der Anker in der Tauchspule dann hin- und herbewegt werden, um das Ein- und Ausschwenken des optischen Bauelements 24 zu bewirken.

Bei dieser Ausgestaltung des Betätigungsmechanismus ist ein äußerer Magnet nicht erforderlich, so daß die Vorrichtung radial besonders schmalbauend ausgebildet werden kann. Im Innern des Gehäuses 22 kann auch eine Doppelspule angeordnet sein, die je eine Wicklung für jede Stellung des optischen Bauelements 24 aufweist, die zum Ein- und Ausschwenken des Bauelements 24 dann entsprechend strombeaufschlagt werden.

Der Träger 40 weist weiterhin einen Anschlag 76 auf, der an dem ersten Schenkel 42 des Trägers 40 durch eine entsprechende Anlagefläche gebildet wird, die im aus dem Strahlengang ausgeschwenkten Zustand des Bauelements 24 an einem mit dem Gehäuse 22 fest verbundenen Bauteil 77 der Vorrichtung 20 in Anlage kommt. Im in den Strahlengang eingeschwenkten Zustand des Bauelements 24 bildet ferner der zweite Schenkel 44 des Trägers 40 einen Anschlag 78, der mit der Führungshülse 64 in Anlage kommt. Somit sind die beiden Schwenkendstellungen des Bauelements 24 durch die Anschläge 76 und 78 wohl definiert. Insbesondere der Anschlag 78 gewährleistet im in den Strahlengang eingeschwenkten Zustand des Bauelements 24, daß dieses bezüglich der optischen Achse stets die gleiche für den Lichtdurchtritt durch das optische Bauelement 24 erforderliche Stellung einnimmt, so daß unerwünschte Fehlstellungen des Bauelements 24 im optischen System vermieden werden.

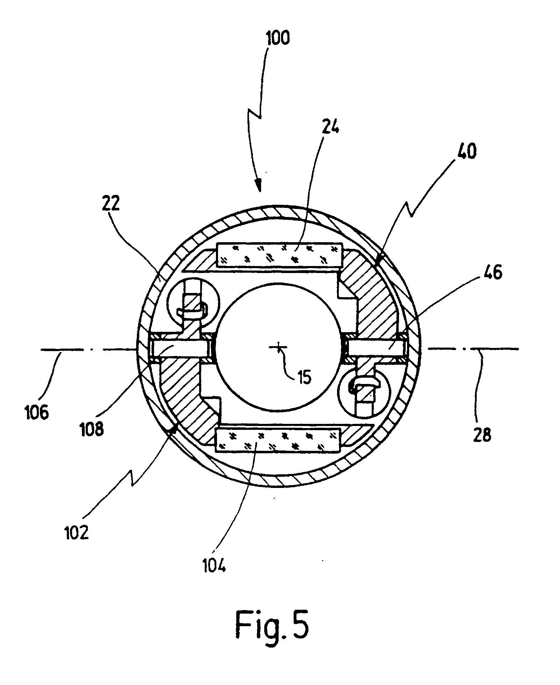

In Fig. 4 ist gemäß einem weiteren Ausführungsbeispiel eine Vorrichtung 80 zum Positionieren mehrerer optischer Bauelemente 82, 84, 86 dargestellt, die ebenfalls in dem Endoskop 10 in Fig. 1 verwendet werden kann. Gleiche Teile wie bei der Vorrichtung 20 sind mit gleichen Bezugszeichen versehen.

Als wesentlichen Unterschied zu der Vorrichtung 20 gemäß Figuren 2 und 3 weist die Vorrichtung 80 einen gegenüber dem Träger 40 modifizierten Träger 88 auf, der die Bauelemente 82, 84, 86 trägt. Während der Träger 88 im Querschnitt wiederum L-förmig ausgebildet ist, trägt der Träger 88 in Schwenkrichtung umfänglich verteilt hier die drei Bauelemente 82, 84, 86, die wahlweise in den Strahlengang des Endoskops 10 eingeschwenkt und aus diesem ausgeschwenkt werden können. Die Bauelemente 82, 84, 86 können bspw. mehrere optische Filter mit unterschiedlichen spektralen Transmissionsgraden oder bspw. ein Farbfilter, eine Linse und ein Wärmeschutzfilter sein.

In der Darstellung gemäß Fig. 4 ist das Bauelement 86 in den Strahlengang des Endoskops 10 eingeschwenkt. Durch Bewegen des äußeren Magneten 56 in Richtung des Pfeiles 72 wird das Bauelement 86 in Richtung eines Pfeiles 90 aus dem Strahlengang ausgeschwenkt, wodurch automatisch damit gekoppelt das Bauelement 84 in den Strahlengang eingeschwenkt wird. Beim weiteren Bewegen des äußeren Magneten 56 in Richtung des Pfeiles 72 wird dann das Bauelement 84 wieder aus dem Strahlengang ausgeschwenkt, während das Bauelement 82 bei dieser Bewegung in den Strahlengang eingeschwenkt wird. Mit einer derartigen Ausgestaltung der Vorrichtung 80 kann somit wahlweise eines der Bauelemente 82, 84, 86 in den Strahlengang eingeschwenkt werden.

In Fig. 5 ist schließlich noch ein weiteres Ausführungsbeispiel einer Vorrichtung 100 zur Verwendung in dem Endoskop 10 dargestellt. Die Vorrichtung 100 stellt eine Modifikation der Vorrichtung 20 gemäß Figuren 2 und 3 dar, wobei wiederum mit der Vorrichtung 20 gleiche Teile mit gleichen Bezugszeichen versehen wurden.

Zusätzlich zu dem Träger 40, der das Bauelement 24 trägt, weist die Vorrichtung 100 einen zweiten Träger 102 auf, der ein weiteres Bauelement 104 trägt. Der Träger 40 und der Träger 102 sind identisch zueinander ausgebildet, jedoch spiegelsymmetrisch zueinander angeordnet. Der Träger 102 und damit das weitere Bauelement 104 ist um eine weitere Schwenkachse 106, die schräg zur optischen Achse 15 verläuft, in den Strahlengang des Endoskops 10 ein- und ausschwenkbar. Die Schwenkachse 106 wird durch einen Gelenkzapfen 108 gebildet.

In dem gezeigten Ausführungsbeispiel gemäß Fig. 5 sind der Träger 40 und der Träger 102 axial an der gleichen Position angeordnet, so daß das Bauelement 24 und alternativ dazu das Bauelement 104 in den Strahlengang eingeschwenkt werden können. Zum Verschwenken des Trägers 102 kann wiederum eine Magnetkupplung vorgesehen sein, wobei dem äußeren Magneten 56 und dem inneren magnetisch wirksamen Element 58 gemäß Fig. 3a) diametral gegenüberliegend ein weiterer äußerer Magnet und weiteres magnetisch wirksames Element angeordnet sein können. Die Magnetkupplung kann jedoch auch so ausgestaltet sein, daß der äußere Magnet 56 gemäß Fig. 3a) auf der Außenseite des Gehäuses 22 nicht nur axial verschieblich, sondern auch verdrehbar angeordnet ist, so daß zum Verschwenken des Trägers 102 nur ein zusätzliches inneres magnetisch wirksames Element vorgesehen werden muß, wobei der äußere Magnet 56 durch Drehen um 180° wahlweise dann mit dem inneren Element 58 oder dem für den Träger 102 vorgesehenen inneren Element magnetisch in Wirkverbindung gebracht werden kann.

Ausgehend von Fig. 5 kann es auch vorgesehen sein, den Träger 102 um seine Schwenkachse 106 gegenüber dem Träger 40 um 90° verschwenkt anzuordnen und den Gelenkzapfen 108 des Trägers 102 mit dem Gelenkzapfen 46 des Trägers 40 starr zu koppeln, so daß die Magnetkupplung 54 gemäß Fig. 3a) mit nur einem äußeren Magneten 56 und nur einem inneren Element 58 ausreicht, um abwechselnd das Bauelement 24 oder das Bauelement 104 in den Strahlengang einzuschwenken und aus dem Strahlengang wieder auszuschwenken.

Anstatt den Träger 102 und den Träger 40 axial an gleicher Position in dem Gehäuse 22 anzuordnen, kann es auch vorteilhaft sein, diese axial an unterschiedlichen Positionen anzuordnen und unabhängig voneinander verschwenkbar um die Schwenkachse 28 bzw. um die Schwenkachse 106 auszugestalten. In diesem Fall können dann das Bauelement 24 und das Bauelement 104 gemeinsam in den Strahlengang eingeschwenkt werden. Bspw. kann das Bauelement 24 dann ein Farbfilter und das Bauelement 104 ein Wärmeschutzfilter, eine Blende oder eine Linse sein.

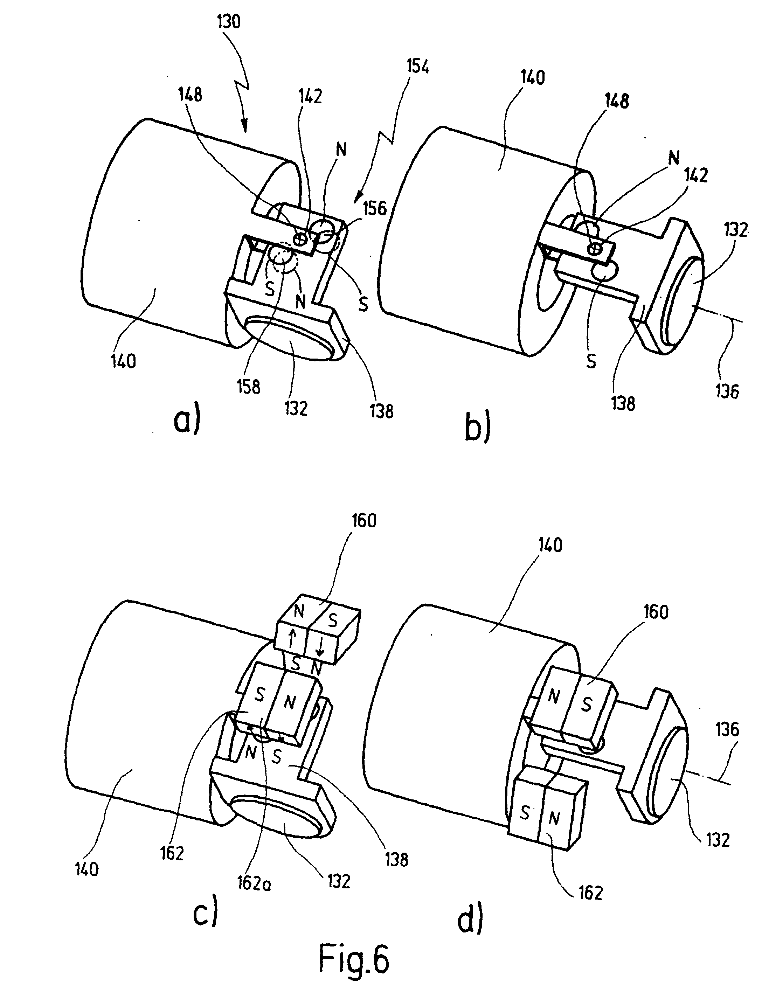

In Fig. 6a) bis d) ist ein weiteres Ausführungsbeispiel einer mit dem allgemeinen Bezugszeichen 130 versehenen Vorrichtung zum Positionieren zumindest eines optischen Bauelements 132 dargestellt, die ebenfalls in dem Endoskop 10 Verwendung finden kann.

Das optische Bauelement 132 ist in Fig. 6b) und d) in einer in den Strahlengang des nicht dargestellten Endoskops eingeschwenkten Zustand dargestellt, so daß die mit 136 bezeichnete optische Achse durch das optische Bauelement 132 hindurchgeht.

Das optische Bauelement 132 kann in den Strahlengang des Endoskops ein- und ausgeschwenkt werden.

Das optische Bauelement 132 ist an einem Träger 138 montiert, der an einem Halter 140 mittels eines Stiftes 142 verschwenkbar montiert ist. Der Halter 140 ist in dem nicht dargestellten Endoskop in dessen Optikkopf befestigt.

In mit Fig. 5 vergleichbarer Weise kann der Halter 140 auch zwei Träger halten, um zwei optische Bauelemente verschwenkbar aufzunehmen.

Der Stift 142 definiert eine Schwenkachse 148, um die das optische Bauelement 132 in den Strahlengang des Endoskops 120 einund aus diesem ausschwenkbar ist.

Der Träger 138 ist im Querschnitt L-förmig ausgebildet.

Die Schwenkachse 148 verläuft schräg, gemäß dem in Fig. 6a) und d) dargestellten Ausführungsbeispiel senkrecht zur optischen Achse 136.

Die Vorrichtung 130 zum Positionieren des optischen Bauelements 132 weist ein nicht dargestelltes Gehäuse auf, das gleichzeitig das Gehäuse des Optikkopfes des Endoskops bildet. Das Gehäuse ist wiederum hermetisch dicht.

Zum Ein- und Ausschwenken des optischen Bauelements 132 ist eine Magnetkupplung 154 vorgesehen. Die Magnetkupplung 154 unterscheidet sich von der Magnetkupplung 54 gemäß Fig. 3 dadurch, daß ein Kraftübertragungselement, wie das Kraftübertragungselement 68 in Fig. 3a, bei der Magnetkupplung 154 nicht erforderlich ist. Die Magnetkupplung 154 ist vielmehr so ausgebildet, wie hiernach noch näher beschrieben wird, daß die magnetische Wirkung zumindest eines äußeren Magneten auf den Träger 138 unmittelbar erfolgt, um diesen umzuklappen.

Anhand der Fig. 6a) bis 6d) wird dies veranschaulicht.

Der Träger 138 ist in Fig. 6a) und 6c) in einer Position dargestellt, in der das optische Bauelement 132 aus dem Strahlengang des Endoskops ausgeschwenkt ist, und in Fig. 6b) und 6d) in einer Position, in der das optische Bauelement 132 in den Strahlengang des Endoskops eingeschwenkt ist.

Die Magnetkupplung 154 umfaßt zwei innere magnetisch wirksame Elemente 156 und 158 in Form von Permanentmagneten.

Wie aus Fig. 6a) hervorgeht, sind die magnetisch wirksamen Elemente 156 und 158 jeweils als zylindrische Stabmagneten ausgebildet, die in den Träger 138 eingelassen sind.

Das magnetisch wirksame Element 156 ist dem magnetisch wirksamen Element 158 bezüglich der durch den Stift 142 definierten Schwenkachse 148 diametral gegenüberliegend angeordnet und entgegengesetzt polarisiert.

Dazu ist bei beiden magnetisch wirksamen Elementen 156 bzw. 158 mit N der Nordpol und mit S der Südpol bezeichnet.

Die Magnetkupplung 154 weist weiterhin zumindest einen, im gezeigten Ausführungsbeispiel zwei äußere Magneten 160 und 162 auf, die in Fig. 6a) und b) weggelassen wurden. Die äußeren Magnete 160 und 162 sind jeweils als Magnetpaare ausgebildet. Mit N wurde wiederum der Nordpol und mit S der Südpol der magnetisch wirksamen Magnetelemente dargestellt.

Die äußeren Magnete 160 und 162 sind an einem nicht dargestellten drehbaren Ring befestigt, der das Gehäuse der Vorrichtung 130 umgibt.

Durch Drehen des Rings kann nun wahlweise der äußere Magnet 160 oder der äußere Magnet 162 mit den inneren magnetischen Elementen 156 und 158 magnetisch in Eingriff gebracht werden.

Fig. 6c) zeigt unter Weglassung des drehbaren Rings eine Drehstellung, in der der äußere Magnet 162 mit den magnetisch wirksamen Elementen 156 und 158 magnetisch in Eingriff steht. Bei dieser Konstellation nimmt der Träger 138 eine Position ein, in der der Südpol S des magnetisch wirksamen Elements 158 dem Nordpol des Magnetelements 162a des äußeren Magneten 162 gegenübersteht.

Bleibt der Ring in dieser Drehstellung, ist der Träger 138 mit aus dem Strahlengang ausgeschwenktem optischen Bauelement 132 in der in Fig. 6c) dargestellten Position wegen der magnetisch anziehenden Wirkung zwischen dem Magnetfeld des Magnetelements 162a und dem magnetisch wirksamen Element 158 in dieser Schwenkstellung festgehalten.

Wird der Ring ausgehend von dieser Stellung so weit verdreht, daß der äußere Magnet 160 den magnetisch wirksamen Elementen 156 und 158 gegenübersteht, übt der äußere Magnet 160 im Zusammenhang mit den magnetisch wirksamen Elementen 156 und 158 auf den Träger 138 ein Drehmoment bezüglich der Schwenkachse 148 aus, mit der Wirkung, daß der Träger 138 um die Schwenkachse 148 verschwenkt und seine in Fig. 6d) gezeigte Position einnimmt. In diesem Fall ist das optische Bauelement 132 in den Strahlengang des Endoskops 120 eingeschwenkt.

Durch Zurückdrehen des Rings wird ausgehend von Fig. 6d) wieder die in Fig. 6c) dargestellte Position des Trägers 138 eingestellt. Durch Hin- und Herdrehen des Rings kann somit das Bauelement 132 zwischen der ein- und ausgeschwenkten Stellung umgeklappt werden.

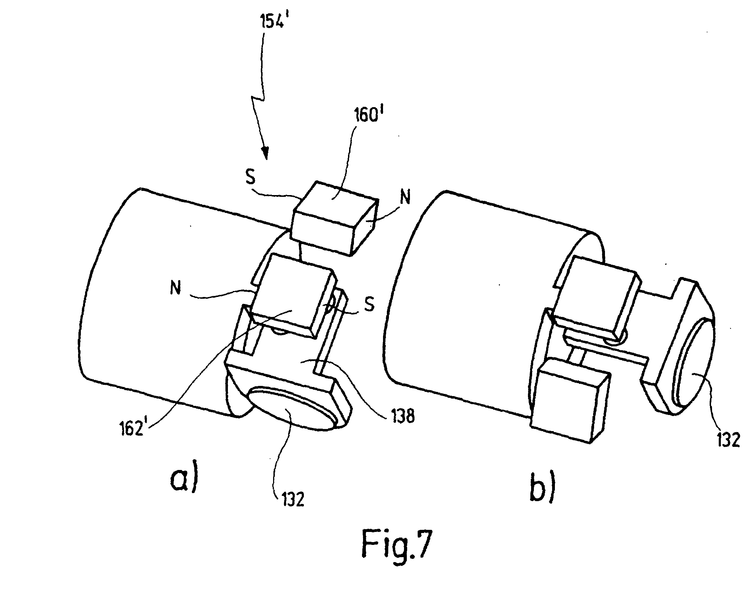

In Fig. 7a) und 7b) ist in einer geringfügigen Abwandlung gegenüber Fig. 6a) bis 6d) dargestellt, daß die äußeren Magnete 160' und 162' nicht als Magnetpaare ausgebildet sind, sondern als Einzelmagnete, wobei wiederum mit S der Südpol und mit N der Nordpol der Magnete 160' bzw. 162' angedeutet ist. Die Positionierung der inneren magnetisch wirksamen Elemente 156 bzw. 158 an dem Träger 138 ist identisch mit derjenigen in Fig. 6a).

Die Funktionsweise der Magnetkupplung 154' ist die gleiche wie diejenige der Magnetkupplung 154.

Ferner ist bei der Vorrichtung 130 vorgesehen, daß der bereits erwähnte Ring, mit dem die äußeren Magnete 160 und 162 verbunden sind, zumindest zwei Raststellungen aufweist, wobei zumindest eine Raststellung einer ausgeschwenkten Stellung des Bauelements 132 und zumindest eine Raststellung einer eingeschwenkten Stellung des Bauelements 132 zugeordnet ist.

Diese verschiedenen Raststellungen werden bspw. durch eine Kugelraste ermöglicht, die in dem Ring angeordnet ist, und mit entsprechenden Rastkerben am Gehäuse zusammenwirkt, die umfänglich verteilt am Gehäuse ausgebildet sind.

In Abwandlungen des Ausführungsbeispiels gemäß Fig. 7 und 8 können die beiden inneren magnetischen Elemente 156 und 158 auch durch einen einzigen Magneten ersetzt werden, bspw. einen Stabmagneten, der entsprechend in dem Träger 138 bzw. 144 positioniert wird.

Auch können die äußeren Magnete 160 und 162 bzw. 160' und 162' durch einen Elektromagneten oder einen Weicheisenkern ersetzt werden, der außerhalb oder innerhalb des Gehäuses 152 angeordnet werden kann.

## Patentansprüche

1. Vorrichtung zum Positionieren zumindest eines optischen Bauelements (24; 82, 84, 86; 104; 132) innerhalb eines endoskopischen Systems, mit einem Gehäuse (22), durch das eine optische Achse (15; 136) des endoskopischen Sytems verläuft und in dem das zumindest eine Bauelement (24; 82, 84, 86; 104; 132) angeordnet ist, das in den Strahlengang des endoskopischen Systems um eine Schwenkachse (28; 106; 148) einschwenkbar und aus dem Strahlengang wieder ausschwenkbar ist, wobei die Schwenkachse (28; 106; 148) schräg oder senkrecht zur optischen Achse (15; 136) verlaufend angeordnet ist, und wobei das zumindest eine Bauelement (24; 82, 84, 86; 104; 132) an einem Träger (40; 138) befestigt ist, der um die Schwenkachse (28; 106; 148) verschwenkbar am Gehäuse (22) befestigt ist, **dadurch gekennzeichnet, daß** der Träger (40; 88; 102; 138) im Querschnitt senkrecht zur optischen Achse (15; 136) etwa L-förmig ausgebildet ist, wobei ein erster Schenkel (42) des Trägers (40; 88; 102; 138) das zumindest eine Bauelement (24; 82, 84, 86; 104; 132) trägt, und ein zweiter Schenkel (44) gelenkig am Gehäuse (22) befestigt ist.

2. Vorrichtung nach Anspruch 1, **dadurch gekennzeichnet, daß** die Schwenkachse (28; 106; 148) senkrecht zur optischen Achse (15; 136) verlaufend angeordnet ist.

3. Vorrichtung nach Anspruch 1 oder 2, **dadurch gekennzeichnet, daß** die Schwenkachse (28; 106; 148) so angeordnet ist, daß das Bauelement (24; 82, 84, 86; 104; 132) im aus dem Strahlengang ausgeschwenkten Zustand mit einer flächigen Seite einer Innenwand (38) des Gehäuses (22) benachbart zu liegen kommt.

4. Vorrichtung nach einem der Ansprüche 1 bis 3, **dadurch gekennzeichnet, daß** in dem Gehäuse (22) zumindest zwei schwenkbare Träger (40; 102) angeordnet sind, die jeweils zumindest ein Bauelement (24; 104) tragen und unabhängig voneinander verschwenkbar sind.

5. Vorrichtung nach Anspruch 4, **dadurch gekennzeichnet, daß** die Träger (40; 102) miteinander derart gekoppelt sind, daß beim Einschwenken des zumindest einen Bauelements (24) das zumindest eine andere Bauelement (104) ausgeschwenkt wird und umgekehrt.

6. Vorrichtung nach Anspruch 4 oder 5, **dadurch gekennzeichnet, daß** die Träger (40; 102), bezogen auf die Längsrichtung des Gehäuses (22), an einer etwa gleichen Position angeordnet sind.

7. Vorrichtung nach Anspruch 4 oder 5, **dadurch gekennzeichnet, daß** die Träger (40; 102), bezogen auf die Längsrichtung des Gehäuses (22), an unterschiedlichen Positionen angeordnet sind.

8. Vorrichtung nach einem der Ansprüche 1 bis 7, **dadurch gekennzeichnet, daß** der Träger (88) in Schwenkrichtung umfänglich verteilt mehrere Bauelemente (24) trägt.

9. Vorrichtung nach einem der Ansprüche 1 bis 8, **dadurch gekennzeichnet, daß** zum Betätigen des Ein- und Ausschwenkens des zumindest einen Bauelements (24; 132) eine Magnetkupplung (54; 154) vorgesehen ist, die zumindest ein außerhalb des Gehäuses (22) angeordnetes äußeres bewegliches magnetisch wirksames Element oder zumindest einen Magneten (56; 160, 162; 160', 162') und zumindest ein innerhalb des Gehäuses (22) angeordnetes inneres magnetisch wirksames Element (58; 156, 158) aufweist, wobei das äußere magnetisch wirksame Element oder der äußere Magnet (56; 160, 162; 160', 162') und das innere magnetisch wirksame Element (58; 156, 158) durch das Gehäuse (22) hindurch über einen magnetischen Kraftschluß zusammenwirken.

10. Vorrichtung nach einem der Ansprüche 1 bis 9, **dadurch gekennzeichnet, daß** der Träger (40; 88; 102) als bezüglich der Schwenkachse (28; 106) zweiarmiger Hebel ausgebildet ist, dessen einer Hebelarm (65) das zumindest eine Bauelement (24; 82, 84, 86; 104) trägt, und an dessen anderem Hebelarm (66) ein im wesentlichen axial bewegliches Kraftübertragungselement (68) angreift.

11. Vorrichtung nach Anspruch 9 und 10, **dadurch gekennzeichnet, daß** das zumindest eine magnetisch wirksame Element oder der äußere Magnet (56) und das zumindest eine innere magnetisch wirksame Element (58) axial verschieblich sind, wobei der innere Magnet (58) mit dem Kraftübertragungselement (68) verbunden ist.

12. Vorrichtung nach Anspruch 9 und 10, **dadurch gekennzeichnet, daß** die Magnetkupplung zumindest zwei äußere magnetisch wirksame Elemente oder Magnete aufweist, die an axial unterschiedlichen Positionen an einem drehbaren Ring angeordnet sind, wobei das zumindest eine innere magnetisch wirksame Element (58) axial verschieblich und mit dem Kraftübertragungselement verbunden ist, und wobei die zwei äußeren magnetisch wirksamen Elemente oder Magnete durch Drehen des Rings alternativ mit dem inneren Element (58) magnetisch in Eingriff bringbar sind.

13. Vorrichtung nach einem der Ansprüche 1 bis 8 und 9, **dadurch gekennzeichnet, daß** das zumindest eine innere magnetisch wirksame Element (156, 158) am Träger (138) selbst angeordnet ist und mit dem zumindest einen äußeren magnetisch wirksamen Element oder Magneten (160, 162; 160', 162') unmittelbar zum Ein- und Ausschwenken des zumindest einen Bauelements (132) zusammenwirkt.

14. Vorrichtung nach Anspruch 13, **dadurch gekennzeichnet, daß** das zumindest eine äußere magnetisch wirksame Element oder Magnet (160, 162; 160', 162') über einen das Gehäuse umgebenden drehbaren Ring beweglich ist.

15. Vorrichtung nach Anspruch 13 oder 14, **dadurch gekennzeichnet, daß** zwei innere magnetisch wirksame Elemente (156, 158) in Form von zwei Magneten am Träger angeordnet sind, wobei die inneren Magnete bezüglich der Schwenkachse (148) einander gegenüberliegend angeordnet und entgegengerichtet polarisiert sind, und daß zumindest zwei äußere Magnete (160, 162; 160', 162') außerhalb des Gehäuses angeordnet sind, die wechselweise in eine Stellung bewegbar sind, in der sie mit den inneren Magneten magnetisch zusammenwirken, um das zumindest eine Bauelement (132) ein- bzw. auszuschwenken.

16. Vorrichtung nach Anspruch 12 oder 14, **dadurch gekennzeichnet, daß** der Ring zumindest zwei Raststellungen aufweist, wobei zumindest eine erste Raststellung einer ausgeschwenkten Stellung des Bauelements (132) und zumindest eine zweite Raststellung einer eingeschwenkten Stellung des Bauelements (132) zugeordnet ist.

17. Vorrichtung nach einem der Ansprüche 9 bis 16, **dadurch gekennzeichnet, daß** das zumindest eine äußere magnetisch wirksame Element ein Permanentmagnet oder ein Weicheisenkern ist.

18. Vorrichtung nach einem der Ansprüche 9 bis 16, **dadurch gekennzeichnet, daß** das zumindest eine äußere magnetisch wirksame Element ein Elektromagnet ist, der auch innerhalb des Gehäuses (22) angeordnet sein kann, wobei das zumindest eine innere magnetisch wirksame Element (58; 156, 158) gegebenenfalls mit einer Rückholfeder verbunden ist.

19. Vorrichtung nach einem der Ansprüche 9 bis 18, **dadurch gekennzeichnet, daß** das zumindest eine innere magnetisch wirksame Element (58; 156, 158) ein Magnet oder ein Weicheisenkern ist.

20. Vorrichtung nach einem der Ansprüche 1 bis 8 und nach Anspruch 10, **dadurch gekennzeichnet, daß** im Inneren des Gehäuses (22) ein Tauchspulantrieb mit einer Elektrospule und einem darin axial beweglichen Anker angeordnet ist, wobei der Anker mit dem Kraftübertragungselement (68) verbunden ist.

21. Endoskop, insbesondere für die photodynamische Diagnose, Therapie oder die Fluoreszenzdiagnose, **gekennzeichnet durch** eine Vorrichtung (20; 80; 100; 130) nach einem der Ansprüche 1 bis 20.

22. Endoskop nach Anspruch 21, **dadurch gekennzeichnet, daß** die Vorrichtung (20; 80; 100; 130) am proximalen Ende des Endoskops (10) in einem Optikkopf (16) zwischen der Okularlinse und dem Deckglas des Okulars angeordnet ist.

23. Endoskop nach Anspruch 21 oder 22, **dadurch gekennzeichnet, daß** das Gehäuse (22) der Vorrichtung (10; 80; 100; 130) das Gehäuse des Endoskops (10) bildet.

24. Endoskop nach Anspruch 23, **dadurch gekennzeichnet, daß** das Gehäuse (22) hermetisch dicht ist.

## Claims

1. A device for positioning at least one optical component (24; 82, 84, 86; 104; 132) within an endoscopic system, comprising a housing (22), through which runs an optical axis (15; 136) of the endoscopic system and in which the at least one component (24; 82, 84, 86; 104; 132) is arranged, which can be pivoted into the beam path of the endoscopic system about a pivot axis (28; 106; 148) and which can be pivoted out of the beam path, wherein the pivot axis (28; 106; 148) is arranged obliquely or orthogonal with respect to the optical axis (15; 136), and wherein the at least one component (24; 82, 84, 86; 104; 132) is fixed on a carrier (40; 138), which is fixed at the housing (22) pivotably about the pivot axis (28; 106; 148), **characterized in that** the carrier (40; 88; 102; 138), in cross-section perpendicular with respect to the optical axis (15; 136), is configured approximately in L-shape, wherein a first leg (42) of the carrier (40; 88; 102; 138) carries the at least one component (24; 82, 84, 86; 104; 132), and a second leg is articulatedly fixed at the housing (22).

2. The device of claim 1, **characterized in that** the pivot axis (28; 106; 148) is arranged orthogonal with respect to the optical axis (15; 136).

3. The device of claim 1 or 2, **characterized in that** the pivot axis (28; 106; 148) is arranged in such a way that the component (24; 82, 84, 86; 104; 132) in the state pivoted out of the beam path comes in an adjacent position with a flat side of an inner wall (38) of the housing (22).

4. The device of anyone of claims 1 through 3, **characterized in that** in the housing (22) at least two pivotable carriers (40; 102) are arranged, which each carry at least one component (24; 104), and which are pivotable independently of each other.

5. The device of claim 4, **characterized in that** the carriers (40; 102) are coupled with each other in such a way that, when the at least one component (24) is pivoted in, the at least one other component (104) is pivoted out, and vice versa.

6. The device of claim 4 or 5, **characterized in that** the carriers (40; 102) are arranged axially in an approximately same position with respect to the longitudinal direction of the housing (22).

7. The device of claim 4 or 5, **characterized in that** the carriers (40; 102) are arranged at axially different positions with respect to the longitudinal direction of the housing (22).

8. The device of anyone of claims 1 through 7, **characterized in that** the carrier (88) carries several components which are distributed in pivot direction over the circumference.

9. The device of anyone of claims 1 through 8, **characterized in that** for actuating the pivoting in and the pivoting out of the at least one component (24; 132) a magnetic coupling (54; 154) is provided, which has at least one outer movable magnetically active element arranged outside the housing (22) or at least one magnet (56; 160, 162; 160', 162') and at least one inner magnetically active element (58; 156, 158) arranged within the housing (22), wherein the outer magnetically active element or the outer magnet (56; 160, 162; 160', 162') and the inner magnetically active element (58; 156, 158) coact through the housing (22) via a magnetic force.

10. The device of anyone of claims 1 through 9, **characterized in that** the carrier (40; 88; 102) is configured as a two-armed lever with respect to the pivot axis (28; 106), the one lever arm (65) of which carries the at least one component (24; 82, 84, 86; 104), and on the other lever arm (66) of which engages an essentially axially movable force transmission element (68).

11. The device of claims 9 and 10, **characterized in that** the at least one magnetically active element or the outer magnet (56) and the at least one inner magnetically active element (58) are axially displaceable, wherein the inner magnet (58) is connected to the force transmission element (68).

12. The device of claims 9 and 10, **characterized in that** the magnetic coupling comprises at least two outer magnetically active elements or magnets, which are arranged on a rotatable ring, in axially different positions, wherein the at least one inner magnetically active element (58) is axially movable and connected to the force transmission element, and wherein the two outer magnetically active elements or magnets can alternatively be brought in magnetic engagement with the inner element (58) by rotating the ring.

13. The device of anyone of claims 1 through 8 and 9, **characterized in that** the at least one inner magnetically active element (156, 158) is arranged on the carrier (138) itself and coacts directly with the at least one outer magnetically active element or magnet (160, 162; 160', 162') for pivoting in and for pivoting out of the at least one component (132).

14. The device of claim 13, **characterized in that** the at least one outer magnetically active element or magnet (160, 162; 160', 162') is movable via a rotatable ring surrounding the housing.

15. The device of claim 13 or 14, **characterized in that** two inner magnetically active elements (156, 158) are arranged, in the form of two magnets, on the carrier, wherein the inner magnets, with respect to the pivot axis (148), are arranged opposite to each other and polarized opposite to each other, and that at least two outer magnets (160, 162; 160', 162') are arranged outside the housing, which are, alternately, movable into a position, in which they coact magnetically with the inner magnets, in order to pivot in or to pivot out the at least one component (132).

16. The device of claim 12 or 14, **characterized in that** the ring has at least two lock-in positions, wherein at least a first lock-in position is assigned to a pivoted-out position of the component (132) and at least a second lock-in position is assigned to a pivoted-in position of the component (132).

17. The device of anyone of claims 9 through 16, **characterized in that** the at least one outer magnetically active element is a permanent magnet or a soft-iron core.

18. The device of anyone of claims 9 through 16, **characterized in that** the at least one magnetically active element is an electric magnet, which can also be arranged within the housing (22), wherein the at least one inner magnetically active element (58; 156, 158) may be connected to a back-force spring.

19. The device of anyone of claims 9 through 18, **characterized in that** the at least one inner magnetically active element (58; 156, 158) is a magnet or a soft-iron core.

20. The device of anyone of claims 1 through 8 and of claim 10, **characterized in that** a moving coil drive with an electric coil and an anchor is arranged in the inner part of the housing (22), the anchor being arranged therein axially movable and being connected to the force transmission element (68).

21. An endoscope, in particular for photodynamical diagnosis or therapy or for fluorescence diagnosis, **characterized by** a device (20; 80; 100; 130) of anyone of claims 1 through 20.

22. The endoscope of claim 21, **characterized in that** the device (20; 80; 100; 130) is arranged at the proximal end of the endoscope (10) in an optical head (16) between the eyepiece lens and the cover glass of the eyepiece.

23. The endoscope of claim 21 or 22, **characterized in that** the housing (22) of the device (10; 80; 100; 130) forms the housing of the endoscope (10).

24. The endoscope of claim 23, **characterized in that** the housing (22) is hermetically tight.

## Revendications

1. Dispositif pour le positionnement d'au moins un élément optique (24 ; 82, 84, 86 ; 104 ; 132) à l'intérieur d'un système endoscopique, avec un boîtier (22), dans lequel passe un axe optique (15 ; 136) du système endoscopique et dans lequel est disposé au moins un composant (24 ; 82, 84, 86 ; 104 ; 132), qui peut être rentré par basculement dans la trajectoire du faisceau du système endoscopique autour d'un axe de pivotement (28 ; 106 ; 148) et peut être de nouveau sorti par basculement de la trajectoire du faisceau, l'axe de pivotement (28 ; 106 ; 148) étant disposé obliquement ou perpendiculairement à l'axe optique (15 ; 136), et au moins un composant (24 ; 82, 84, 86 ; 104 ; 132) étant fixé sur un support (40 ; 138), qui est fixé sur le boîtier (22) de façon à pouvoir basculer autour de l'axe de pivotement (28 ; 106 ; 148), **caractérisé en ce que** le support (40 ; 88 ; 102 ; 138) est réalisé sensiblement en forme de L vu en coupe perpendiculairement à l'axe optique (15 ; 136), une première branche (42) du support (40 ; 88 ; 102 ; 138) portant le au moins un composant (24 ; 82, 84, 86 ; 104 ; 132) et une seconde branche (44) étant fixée de façon articulée sur le boîtier (22).

2. Dispositif selon la revendication 1, **caractérisé en ce que** l'axe de pivotement (28; 106; 148) est disposé perpendiculairement à l'axe optique (15 ; 136).

3. Dispositif selon la revendication 1 ou 2, **caractérisé en ce que** l'axe de pivotement (28 ; 106 ; 148) est disposé de telle sorte que le composant (24 ; 82, 84, 86 ; 104 ; 132) est au même niveau et voisin avec un côté plan d'une paroi interne (38) du boîtier (22) dans l'état basculé hors de la trajectoire du faisceau.

4. Dispositif selon l'une des revendications 1 à 3, **caractérisé en ce que** dans le boîtier (22) sont disposés au moins deux supports basculants (40; 102) qui supportent chacun au moins un composant (24; 104) et peuvent basculer indépendamment l'un de l'autre.

5. Dispositif selon la revendication 4, **caractérisé en ce que** les supports (40 ; 102) sont couplés l'un avec l'autre de telle sorte que, lors du basculement vers l'intérieur du au moins un composant (24), le au moins un autre composant (104) est basculé vers l'extérieur et inversement.

6. Dispositif selon la revendication 4 ou 5, **caractérisé en ce que** les supports (40 ; 102) sont disposés sur une position sensiblement identique par rapport au sens longitudinal du boîtier (22).

7. Dispositif selon la revendication 4 ou 5, **caractérisé en ce que** les supports (40 ; 102) sont disposés en différentes positions par rapport au sens longitudinal du boîtier (22).

8. Dispositif selon l'une des revendications 1 à 7, **caractérisé en ce que** le support (88) porte plusieurs composants (24) repartis à la périphérie dans le sens de basculement.

9. Dispositif selon l'une quelconque des revendications 1 à 8, **caractérisé en ce que**, pour la commande du basculement vers l'intérieur et vers l'extérieur du au moins un composant (24 ; 132), il est prévu un accouplement magnétique (54 ; 154), qui présente au moins un élément actif magnétiquement, mobile, extérieur, disposé en dehors du boîtier, ou au moins un élément (58 ; 156, 158) actif magnétique, intérieur et disposé à l'intérieur du boîtier (22), l'élément extérieur, actif magnétique, ou l'élément (56; 160, 162; 160', 162') extérieur et l'élément (58 ; 156, 158) intérieur et actif magnétique coopèrent à travers le boîtier (22) au moyen d'une adhérence magnétique.

10. Dispositif selon l'une des revendications 1 à 9, **caractérisé en ce que** le support (40 ; 88 ; 102) est conçu comme un levier à deux bras par rapport à l'axe de pivotement (28 ; 106), dont un bras de levier (65) porte le au moins un composant (24 ; 82, 84, 86 ; 104), et sur l'autre bras de levier (66) duquel est appliqué un élément de transmission de force (68) mobile essentiellement dans le sens axial.

11. Dispositif selon les revendications 9 et 10, **caractérisé en ce que** le au moins un élément actif magnétiquement ou l'aimant (56) extérieur et le au moins un élément (58) intérieur et actif magnétiquement sont coulissants dans le sens axial, l'aimant (58) intérieur étant relié à l'élément de transmission de force (68).

12. Dispositif selon les revendications 9 et 10, **caractérisé en ce que** l'accouplement magnétique présente au moins deux éléments ou aimants extérieurs et actifs magnétiquement, qui sont disposés sur une bague tournante en des positions différentes dans le sens axial, le au moins un élément (58) intérieur et actif magnétiquement étant coulissant axialement et relié à l'élément de transmission de force, et les deux éléments ou aimants extérieurs et actifs magnétiquement pouvant être amenés en coopération magnétiquement au choix avec l'élément (58) intérieur par rotation de la bague.

13. Dispositif selon l'une des revendications 1 à 8 et 9, **caractérisé en ce que** le au moins un élément (156, 158) intérieur et actif magnétiquement est disposé sur le support (138) même, et coopère avec le au moins un élément ou aimant (160, 162 ; 160', 162') extérieur et actif magnétiquement directement pour le basculement vers l'intérieur et l'extérieur du au moins un composant (132).

14. Dispositif selon la revendication 13, **caractérisé en ce que** le au moins un élément ou aimant (160, 162 ; 160', 162') extérieur et actif magnétiquement est mobile au moyen d'une bague rotative et entourant le boîtier.

15. Dispositif selon la revendication 13 ou 14, **caractérisé en ce que** deux éléments (156, 158) intérieurs et actifs magnétiquement sont disposés sous la forme de deux aimants sur le support, les deux aimants intérieurs étant disposés l'un en face de l'autre par rapport à l'axe de pivotement (148) et étant polarisés dans le sens contraire, et **en ce qu'**au moins deux aimants (160, 162 ; 160', 162') extérieurs sont disposés à l'extérieur du boîtier, qui peuvent être amenés à tour de rôle dans une position, dans laquelle ils coopèrent magnétiquement avec l'aimant intérieur, afin de basculer au moins un composant (132) vers l'intérieur ou l'extérieur.

16. Dispositif selon la revendication 12 ou 14, **caractérisé en ce que** la bague présente au moins deux positions d'arrêt, au moins une première position d'arrêt étant associée à une position basculée vers l'extérieur du composant (132) et au moins une seconde position d'arrêt étant associée à une position basculée vers l'intérieur du composant (132).

17. Dispositif selon l'une des revendications 9 à 16, **caractérisé en ce que** le au moins un élément extérieur et actif magnétiquement est un aimant permanent ou un noyau en fer doux.

18. Dispositif selon l'une des revendications 9 à 16, **caractérisé en ce que** le au moins un élément extérieur et actif magnétiquement est un électroaimant, qui peut être disposé également à l'intérieur du boîtier (22), le au moins un élément (58 ; 156, 158) intérieur et actif magnétiquement étant relié éventuellement à un ressort de rappel.

19. Dispositif selon l'une des revendications 9 à 18, **caractérisé en ce que** le au moins un élément (58; 156, 158) intérieur et actif magnétiquement est un aimant ou un noyau en fer doux.

20. Dispositif selon l'une des revendications 1 à 8 et selon la revendication 10, **caractérisé en ce qu'**une commande à bobine plongeuse avec une bobine électrique et un induit mobile axialement à l'intérieur est disposée à l'intérieur du boîtier (22), l'induit étant relié à l'élément de transmission de force (68).

21. Endoscope, en particulier pour le diagnostic photodynamique, la thérapie ou le diagnostic par fluorescence, **caractérisé par** un dispositif (20 ; 80 ; 100 ; 130) selon l'une des revendications 1 à 20.

22. Endoscope selon la revendication 21, **caractérisé en ce que** le dispositif (20 ; 80 ; 100 ; 130) est disposé sur l'extrémité proximale de l'endoscope (10) sur une tête d'optique (16) entre la lentille oculaire et le verre de recouvrement de l'oculaire.

23. Endoscope selon la revendication 21 ou 22, **caractérisé en ce que** le boîtier (22) du dispositif (10 ; 80 ; 100 ; 130) forme le boîtier de l'endoscope (10).

24. Endoscope selon la revendication 23, **caractérisé en ce que** le boîtier (22) est hermétiquement étanche.
